# EUROPEAN PATENT SPECIFICATION

(11) **EP 3 455 339 B1**
(45) Date of publication and mention of the grant of the patent: **25.11.2020**
(21) Application number: 17725083.4
(22) Date of filing: 11.05.2017
(51) Int. Cl.: C12M 1/00, C12M 1/04, C12M 1/06

(54) **STRUCTURED BAG FOR CELL CULTURE**
STRUKTURIERTER ZELLKULTURBEUTEL
POCHE DE CULTURE CELLULAIRE STRUCTURÉE

(30) Priority: 11.05.2016 US 201662334793 P; 06.09.2016 US 201662383743 P
(43) Date of publication of application: 20.03.2019
(73) Proprietor: Corning Incorporated, Corning, New York 14831 (US)
(72) Inventor: MARTIN, Gregory Roger, Acton, ME 04001 (US); TANNER, Allison Jean, Portsmouth, NH 03801 (US)
(74) Representative: Elkington and Fife LLP
(86) International application number: PCT/US2017/032220
(87) International publication number: WO 2017/197154

(56) References cited:
- WO-A1-2016/069892
- WO-A2-2011/102931
- JP-A- 2002 101 867
- DATABASE WPI Week 200257 2 October 2000 (2000-10-02) Thomson Scientific, London, GB; AN 2002-531593 XP002772546, -& JP 2002 101867 A (FUJIMORI IND CO LTD) 9 April 2002 (2002-04-09)
- Thermo Scientific: "BPC Chamber selection guide", , 2015, XP002772502, Retrieved from the Internet: URL:https://assets.thermofisher.com/TFS-As sets/LSG/brochures/Chamber-Selection-Guide -v2-0215-FLR.pdf [retrieved on 2017-07-24]

## Description

### FIELD

The present disclosure generally relates to bags for cell culture and systems employing the same. In particular, the present disclosure relates to structured bags having features that permit the bags to be free standing.

### BACKGROUND

Bags containing fluids under highly sterile conditions are used in the bioprocessing industry for the formulation, storage, transfer and transport of fluid while maintaining sterile conditions. Some of the characteristics of the bags to preserve the quality of the products contained within include biocompatibility with the products, sterility, and non-pyrogenicity. The bags are typically disposed of after use and are recognized as efficient means to prepare and store sterile fluids. Generally, these disposable bioprocessing bags are flexible and made from compatible plastic that is sterilized by Gamma radiation. The bags can be used for all bioprocessing applications including, but not limited to, formulating, filing, storing and transporting final product, stocking pharmaceuticals in cold storage or deep freeze and finally for sampling and analytical purposes. Additionally, the bags may be used for biological fluids such as serum, buffers, and ultrapure water and also for growing cell cultures to obtain the valuable biopharmaceutical compounds produced by cells.

Bioprocessing bags typically include a single material or a laminate of materials folded or cut and sealed to provide a container or vessel to hold medium and cells. The bags are generally flexible and disposable. Many are made from gas permeable materials such as fluorinated ethylene propylene (FEP) copolymers (such as VueLife® culture bags commercially available from American Fluoroseal, Gaithersburg, Maryland and PermaLife bags commercially available from OriGen Biomedical, Austin, Texas) or a styrene-polyolefin laminate (such as LIFECELL bags commercially available from Baxter, Deerfield, Illinois). These bags are commonly configured to be supported in rigid containers and are provided with features that cooperate with the rigid container to hold a shape of the bag such that the internal volume of the bag is maximized. The bags are not structured and do not hold their shape when the bags are placed on a surface.

WO 2016/069892 A1 discloses apparatuses for culturing cells.

JP 2002101867 A discloses a self-standing package bag.

### SUMMARY

According to a first aspect of the present invention, there is provided a structured bag for cell culture as defined in claim 1.

Additional features and advantages will be set forth in the detailed description which follows, and in part will be readily apparent to those skilled in the art from that description or recognized by practicing the embodiments as described herein, including the detailed description which follows, the claims, as well as the appended drawings.

It is to be understood that both the foregoing general description and the following detailed description are merely exemplary, and are intended to provide an overview or framework to understanding the nature and character of the claims. The accompanying drawings are included to provide a further understanding, and are incorporated in and constitute a part of this specification. The drawings illustrate one or more embodiment(s), and together with the description serve to explain principles and operation of the various embodiments.

### BRIEF DESCRIPTION OF THE DRAWINGS

The disclosure will be understood more clearly from the following description and from the accompanying figures, given purely by way of non-limiting example, in which:
FIG. 1A is a front view of a structured bag for cell culture according to embodiments of the present disclosure;
FIG. 1B is a side view of a structured bag for cell culture according to embodiments of the present disclosure;
FIG. 2 illustrates a structured bag for cell culture;
FIG. 3 illustrates a perfusion system including a structured bag in accordance with embodiments of the present disclosure;
FIG. 4 illustrates a circulation system including a structured bag in accordance with embodiments of the present disclosure;
FIG. 5 illustrates a disposable spinner flask including a structured bag in accordance with embodiments of the present disclosure;
FIG. 6A illustrates a structured bag including a porous support in accordance with embodiments of the present disclosure;
FIG. 6B illustrates a structured bag including a porous support in accordance with embodiments of the present disclosure;
FIG. 7 illustrates a structured bag including a porous support in accordance with embodiments of the present disclosure;
FIG. 8A illustrates an exemplary support component for a structured bag in accordance with embodiments of the present disclosure;
FIG. 8B illustrates an exemplary support component for a structured bag in accordance with embodiments of the present disclosure;
FIG. 8C illustrates an exemplary support component for a structured bag in accordance with embodiments of the present disclosure; and
FIG. 9 illustrates a disposable spinner flask including a structured bag having a filter material in accordance with embodiments of the present disclosure.

### DETAILED DESCRIPTION

Reference will now be made in detail to the present embodiment(s), an example(s) of which is/are illustrated in the accompanying drawings. Whenever possible, the same reference numerals will be used throughout the drawings to refer to the same or like parts. The present invention is defined by the claims only. In this description the term "embodiment" does not necessarily refer to claimed subject-matter.

The singular forms "a," "an" and "the" include plural referents unless the context clearly dictates otherwise. The endpoints of all ranges reciting the same characteristic are independently combinable and inclusive of the recited endpoint. All references are incorporated herein by reference.

As used herein, "have," "having," "include," "including," "comprise," "comprising" or the like are used in their open ended sense, and generally mean "including, but not limited to."

All scientific and technical terms used herein have meanings commonly used in the art unless otherwise specified. The definitions provided herein are to facilitate understanding of certain terms used frequently herein and are not meant to limit the scope of the present disclosure.

The present disclosure is described below, at first generally, then in detail on the basis of several exemplary embodiments. The features shown in combination with one another in the individual exemplary embodiments do not all have to be realized. In particular, individual features may also be omitted or combined in some other way with other features shown of the same exemplary embodiment or else of other exemplary embodiments.

Embodiments of the present disclosure relate to structured bags for cell culture. As used herein, the term "structured bag" refers to a bag having a specific form which allows the bag to be free standing. This differs from typical cell culture bags that hold fluid and/or cells which are formed from flexible materials and do not include a form that allows the bags to hold their shape when placed on a surface, or, alternatively, which are flat, rectangular, "pillow-style" bags formed by seaming together two flexible sheets. Provided herein are devices and systems including a structured bag configured for cell culture. Structured bags in accordance with embodiments of the present disclosure are formed from disposable materials and may be discarded after a single use, thereby eliminating washing/sterilizing operations as well as maintenance associated with conventional cell culture vessels.

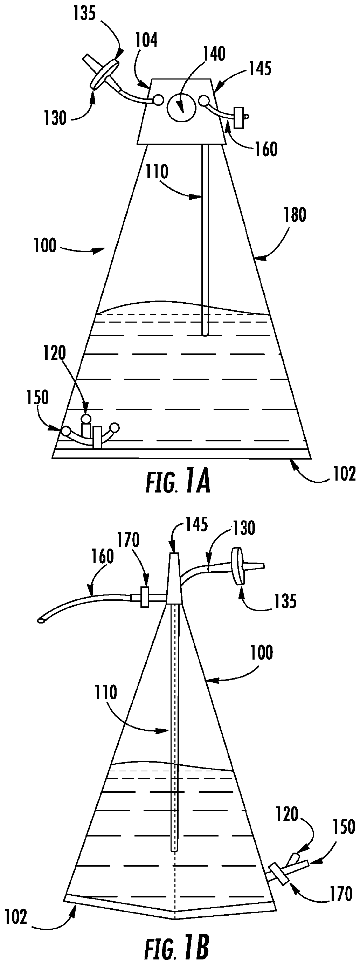

FIGS. 1A-1B illustrate a structured bag for cell culture in accordance with embodiments of the present disclosure. FIG. 1A is a front view of a structured bag 100 and FIG. 1B is a side view of a structured bag **100**. The structured bag **100** includes a top portion **104**, a bottom portion **102** and a sidewall **180** extending between the top portion **104** and the bottom portion **102**. The sidewall **180** at least partially defines an interior compartment for receiving fluid. As will be described in more detail below, the bottom portion **102** has a shape and is configured to maintain the shape to support the structured bag **100** such that the structured bag 100 is free standing when placed on a surface.

As used herein, the term "fluid" is used to denote any substance capable of flowing, such as liquids, liquid suspensions, gases, gaseous suspensions, or the like, without limitation. The term "fluid and/or other components" is used throughout the present disclosure to refer to fluid which may include cell culture media having nutrients for cell growth, cells, byproducts of the cell culture process, and any other biological materials or components that may conventionally be added or formed in a bioprocess system. Structured bags and other vessels described herein may include one or more cells or reagents. Additionally, the bags may include cell culture media. Cell culture media may be for example, but is not limited to, sugars, salts, amino acids, serum (e.g., fetal bovine serum), antibiotics, growth factors, differentiation factors, colorant, or other desired factors. Common culture media that may be provided in the bag includes Dulbecco's Modified Eagle Medium (DMEM), Ham's F12 Nutrient Mixture, Minimum Essential Media (MEM), RPMI Medium, and the like. Any type of cultured cell may be included in the bag including, but not limited to, immortalized cells, primary culture cells, cancer cells, stem cells (e.g., embryonic or induced pluripotent), etc. The cells may be mammalian cells, avian cells, piscine cells, etc. The cells may be of any tissue type including, but not limited to, kidney, fibroblast, breast, skin, brain, ovary, lung, bone, nerve, muscle, cardiac, colorectal, pancreas, immune (e.g., B cell), blood, etc. The cells may be in any cultured form in the bag including disperse (e.g., freshly seeded), confluent, 2-dimensional, 3-dimensional, spheroid, etc. In some embodiments, cells are present without media (e.g., freeze-dried, in preservative, frozen, etc.).

The structured bag **100** may be hermetically sealed and may have one or more openings or fittings for introducing or recovering a fluid. Where the structured bag **100** includes one or more openings, the one or more openings may include seals that in a first configuration expose the one or more openings to fluid communication between the outside of the bag and the interior compartment of the bag through the opening. In a second configuration, the seals close the one or more openings and prevent or reduce fluid communication between the outside of the bag and the interior compartment of the bag through the opening. The seals may take any desired form, including, but not limited to, a clamp, tape, a cap, a zipper, a slide zipper, interlocking or coupling structures, and the like. Alternatively, the structured bag **100** may be unsealed or open-ended. For example, the top portion **104** may be unsealed such that that the structured bag **100** has an interior compartment fluidly connected to the outside of the bag via the unsealed top portion **104**. Optionally, the structured bag **100** may provide a closed system for use in all phases of processing fluid and/or other components. As used herein, the term "closed system" refers to a system sealed to ensure sterility of the contents of the system and to limit or prevent the introduction of contaminants from the surrounding atmosphere. The structured bag **100** may include one or more openings for filling, spiking, adding and/or draining fluid and/or other components. The particular geometry of the structured bag **100** may be determined by particular applications. For example, in the case of a sterile fluid, a hermetically sealed, pre-sterilized bag with an aseptic fitting might be desirable; whereas, in the case where sterility is not important, an open-ended or unsealed bag might be suitable.

According to embodiments of the present disclosure, and as shown for example in FIGS. 1A-1B, the top portion **104** of the structured bag **100** may include a fitment **145**. The fitment **145** may include at least one port. For example, the fitment **145** of FIGS. 1A-1B includes a vent port **130** connected to a vent filter **135** (e.g., 0.2 micrometer vent filter) and an exit port **160** which fluidly connects the interior compartment of the bag to an external vessel (not shown). The vent port **130** may permit exchange of gas between the interior compartment of the bag and outside of the bag, or between at least two bags to decrease or increase air pressure in at least one of the at least two bags. The exit port **160** may optionally fluidly connect a dip tube **110** in the interior compartment of the structured bag **100** to the external vessel. The dip tube **110** may include a proximal end in fluid communication with the exit port **160** and may extend from the exit port **160** into the interior compartment of the bag such that the distal end of the dip tube **110** is positioned to contact fluid occupying the interior compartment of the bag. The fitment **145** may also include a hole **140** which enables the structured bag **100** to be placed on a hook and suspended above a surface.

According to embodiments of the present disclosure, and as shown for example in FIGS. 1A-1B, the bottom portion **102** and/or the sidewall **180** may include at least one port. For example, the sidewall **180** may include a fluid sampling port **150** connected to a "Y" fitting having two arms. One arm of the "Y" may be connected to a media feed vessel (for the addition of fresh medium (not shown)). The other arm **120** of the "Y" is a needleless injection port that may be used for sampling or the addition of cells or other reagents.

While the structured bag **100** is illustrated as having more than one port, embodiments of the present disclosure are not so limited. For example, a single port could be used for both loading and draining of fluid and/or other components from the bag. Ports as described herein may permit transfer of fluid and/or other components from outside of the bag to the interior compartment of the bag, or from the interior compartment of the bag to outside of the bag, or from one bag to another bag (of the same or different type). Additionally, clamps are depicted in the figures by small rectangles **170** attached to tubing at the ports. Alternatively, valves may be used in place of, or in addition to, clamps.

The structured bag **100** may be formed from materials that are conventionally associated with disposable products for bioprocess applications. The sidewall **180** may be formed from a single continuous piece of material. Alternatively, the sidewall **180** may include two or more panels joined along edges thereof. The sidewall **180** and/or the bottom portion **102** and/or the top portion **104** may be formed from a single piece of material whereby boundaries of the various portions of the structured bag **100** are defined by folds or creases. Alternatively, one or more of the side wall **180**, the bottom portion **102** and the top portion **104** may be formed separately and joined along edges thereof.

Any or all of the top portion **104**, the bottom portion **102** and the sidewall **180** may be formed from a film or laminate that includes at least one plastic material from the following group: polyethylene (PE), polypropylene (PP), polyvinyl chloride (PVC), polyethylene terephtalate (PET), polystyrene (PS), polycarbonate (PC), polymethylpentene (PMP), polyetheretherketone (PEEK) polytetrafluoroethylene (PTFE), polyethylene-co-vinyl acetate (EVA), polyfluoroalkoxy (PFA) and derivatives thereof. The film or laminate may have a low melting temperature and preferably exhibits low levels of extractable and leachable components when placed in contact with cell culture fluid and/or other components. The film or laminate may also have high gas permeability.

Optionally, the film or laminate may include at least one layer or coating of graphene, or of a metal such as, but not limited to, stainless steel, nickel, chromium, titanium, molybdenum, tungsten, tantalum, niobium, zirconium, gold, tin, and/or oxides thereof (including alloys thereof), or other metals and/or metal oxides. Non-limiting examples of stainless steel include 200-series, 300-series, 400-series and 500-series, including the grades 201, 202, 301, 302, 303, 304, 305, 308, 309, 310, 314, 316, 316L, 321, 347, 348, 403, 209, 410, 416, 420, 429, 430, 431, 434, 44, 442, 446, 501 and 502. The at least one metallic layer may be included in the film or laminate as a non-contact layer (i.e: the at least one metallic layer does not contact fluid and/or other components in the interior compartment of the bag) and may serve as a gas barrier layer. Alternatively, the at least one metallic layer may be included in the film or laminate as a contact layer (i.e: the at least one metallic layer does not contact fluid and/or other components in the interior compartment of the bag) and may serve as a low extractable and leachable contact layer. The metallic layer may include a metal oxide surface. The metal oxide surface may include the same or a different metal than the metal of the metallic layer. The metallic layer may also include aluminum metal or aluminum metal alloys having a passivating oxide (corrosion resistant) surface layer. In some such embodiments, the metallic layer may be formed from a bio-inert metal and/or metal oxide; where, for example, the metal and/or metal oxide resists corrosion from aldehydes and amines, as may be present in the fluid and/or other components in the interior compartment of the bag. The metal and/or metal oxide may resist corrosion to aqueous buffer solutions (for example phosphate or tris buffers) and/or aqueous saline solutions. As used herein, the term "resists corrosion" refers to the bio-inert metal oxidizes at a rate of less than or equal to 1000 nanometers (nm) depth in 24 hours at 50 degrees Celsius (°C) in deionized water, such as less than or equal to 100 nm depth, and in some embodiments less than or equal to 10 nm depth in 24 hours at 50°C in deionized water.

Each of the top portion **104**, the bottom portion **10**2 and the sidewall **180** may be formed from one or more of the same or different materials. Any or all of the top portion **104,** the bottom portion **102** and the sidewall **180** may be substantially clear to allow for the viewing of fluid and/or other components in the interior compartment of the bag, although the use of opaque or colored materials is also possible, for example where the film or laminate includes a metallic layer. Where opaque or colored materials are used, at least a portion of the sidewall **180** may be substantially clear to allow for viewing of fluid and/or other components in the interior compartment of the bag. Optionally, the sidewall **180** may include volumetric indicia for measuring the volume of the fluid and/or other components in the bag.

Total thickness of the film or laminate may be selected, for example, based on the desired gas permeability of the structured bag **100** or based on the desired rigidity or flexibility of the portion of the bag. For example, the thickness of the film or laminate used to form any of the top portion **104**, the bottom portion **102** and the sidewall **180** may be between about 0.002 inches and about 1.5 inches. The thickness of the flexible material of the bottom portion **102** is thicker than the thickness of the flexible material of at least one of the top portion **104** and the sidewall **180**. As described herein, the thickness of films or laminates used to form the top portion **104,** the bottom portion **102** and the sidewall **180** may be the same or different, provided the thickness of the flexible material of the bottom portion **102** is thicker than at least one of the sidewall **180** and the top portion **104**.

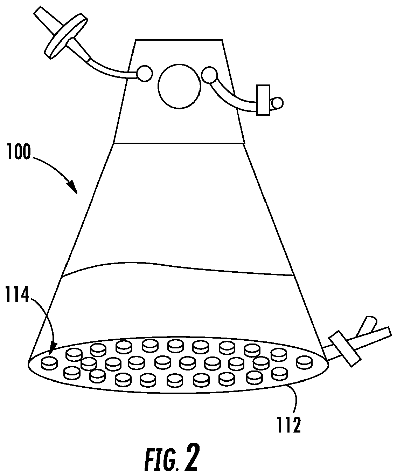

FIG. 2 illustrates a structured bag for cell culture not according to embodiments of the present invention. The structured bag **100** includes a bottom portion **102** formed from a rigid substrate **112**. The rigid substrate **112** may be formed of materials, such as high density polyethylene (HDPE), ultrahigh molecular weight (UHMW) polyethylene, or like materials. While these materials do have some inherent flexibility when used to form relatively thin components or when a moderate amount of bending force is applied thereto, the rigid substrate **112** is distinguished from the flexible portions of the structured bag **100** in that the rigid substrate **112** generally maintains its shape under the weight of fluid and/or other components in the interior compartment of the structured bag **100**. As in the structured bag **100** shown in FIG. 2, the rigid substrate **112** may include geometric patterns **114,** such as microwells shown in FIG. 2, that support three dimensional cell growth, such as cell growth into spheroids or 3D colonies. Exemplary well configurations are described in PCT Patent Application No. PCT/US15/058048. The geometric patterns **114** may form a non-planar surface of the rigid substrate **112** which alters the local fluid-surface interactions and, in turn, promotes mixing of the fluid and/or other components. The geometric patterns **114** may be any shape, for example ridges or undulating features on a surface of the rigid substrate **112**.

The bottom portion **102** of the structured bag **100** may be formed from the same material or from different materials as the other portions of the structured bag **100**. FIG. 2 shows an example where the bottom portion **102** of the structured bag **100** is formed from a different material than the other portions of the bag. While FIG. 2 shows an example where the bottom portion **102** is a rigid material, in embodiments the bottom portion **102** is formed from the same or a different flexible material that is thicker than the flexible materials that form the other portions of the structured bag **100**. The bottom portion **102** may have a circular, ovoid, or polygonal (e.g., triangular, square, rectangular, etc.) shape and may include a bottom panel. The bottom panel may have an outer edge. According to embodiments of the present disclosure, the outer edge of the bottom panel may be formed from the same or different material as the other portions of the bottom panel. For example, the outer edge of the bottom panel may be formed from the same or a different flexible material that is, for example, denser, thicker or heavier than the flexible material of the other portions of the bottom panel. The outer edge of the bottom panel may include one or more weights of any desired composition. For example, the weights may be, but are not limited to, metal, ceramic, plastic, and glass. The weights facilitate maintaining the shape of the bottom portion to support the structured bag **100** such that the structured bag **100** is free standing when placed on a surface.

As illustrated throughout the present disclosure, and in particular in FIGS. 1A-1B, structured bags according to embodiments of the present disclosure may have a broad bottom portion **102**. As used herein, the term "broad bottom portion" is used to describe a bottom portion having at least one dimension that is larger than the same dimension, or a similar dimension, of the top portion. Exemplary dimensions include length, width, diameter, etc. Generally, the bottom portion **102** has a larger area than the top portion **104** (i.e., the cross-sectional area of the bag is reduced from bottom to top). Where the structured bag **100** includes a fitment **145**, the dimensions of the top portion may be defined by the dimensions of the fitment **145**.

It should be understood that the dimensions of the structured bag **100** including both relative and absolute dimensions can be varied. For example, the bags may be configured to hold a volume of fluid and/or other components of about 1.0 ml, or about 5.0 ml, or about 10 ml, or about 25 ml, or about 50 ml, or about 100 ml, or about or about 250 ml, or about 500 ml, or about 1000 ml, or about 2000 ml, or about 5000 ml, or about 10,000 ml, or even about 20,000 ml, as well as all volumes therein between.

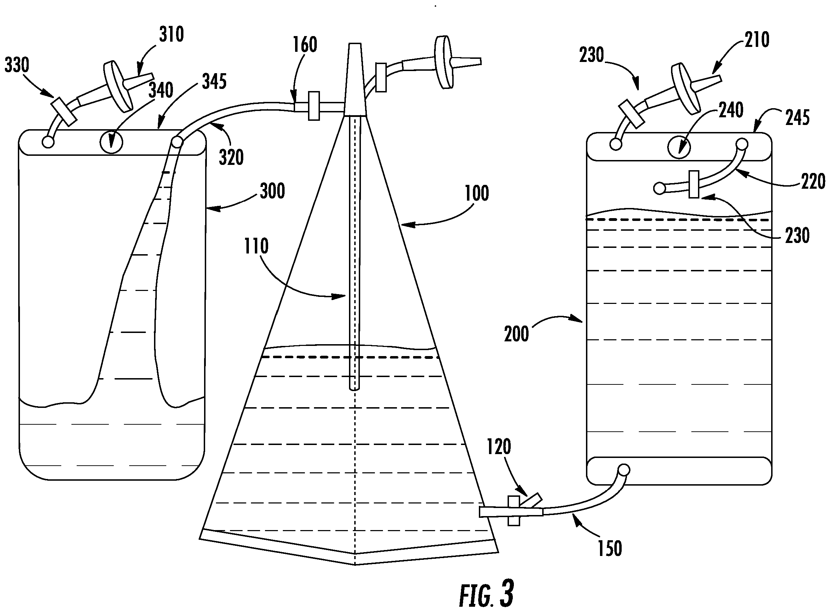

FIG. 3 illustrates a perfusion system including a structured bag in accordance with embodiments of the present disclosure. As shown, a structured bag **100** such as the one shown in FIGS. 1A-1B is placed in fluid communication with a feed vessel **200** and an external vessel **300** that is a different vessel from the feed vessel **200**. The feed vessel **200** and the external vessel **300** may be any type of vessel including any conventional flexible bag, a structured bag such as disclosed herein, or any rigid container, and may be connected and disconnected from the structured bag **100** through any type of connection including, but not limited to, tube-welded connectors, aseptic connectors or other types of connectors.

The feed vessel **200** includes a fitment **245** which may include at least one port. For example, the fitment **245** of FIG. 3 includes a vent port **210** and a feed port **220.** The vent port **210** permits exchange of gas between the interior compartment of the feed vessel **200** and outside of the feed vessel **200**, and the feed port **220** permits addition of fluid and/or other components to the feed vessel **200**. The fitment **245** may also include a hole **240** which enables the feed vessel **200** to be placed on a hook and suspended above a surface. According to embodiments of the present disclosure, and as shown for example in FIG. 3, the bottom and/or the sidewall of the feed vessel **200** may include at least one port. For example, the feed vessel **200** may include a port configured to be fluidly connected to the fluid sampling port **150** of the structured bag **100**, such as through tubing. Where the sidewall of the feed vessel **200** includes a port configured to be fluidly connected to the fluid sampling port **150** of the structured bag **100**, such port is preferably near the bottom of the feed vessel **200**. While the feed vessel **200** is illustrated as having more than one port, embodiments of the present disclosure are not so limited. For example, a single port could be used for both loading and draining of fluid and/or other components from the feed vessel **200**. Ports as described herein may permit transfer of fluid and/or other components from outside of the feed vessel **200** to the interior compartment of the feed vessel **200**, or from the interior compartment of the feed vessel **200** to outside of the feed vessel **200**, or from one feed vessel **200** to another vessel or bag (of the same or different type). Additionally, clamps are depicted in the figures by small rectangles **230** attached to tubing at the ports. Alternatively, valves may be used in place of or in addition to clamps.

The external vessel **300** also includes a fitment **345** which may include at least one port. For example, the fitment **345** of FIG. 3 includes a vent port **310** and a feed port **320.** The vent port **310** permits exchange of gas between the interior compartment of the external vessel **300** and outside of the external vessel **300**, and the feed port **320** may be configured to be fluidly connected to the exit port **160** of the structured bag **100**, such as through tubing. According to embodiments of the present disclosure, the sidewall of the external vessel **300** may include the feed port **320**. For example, the feed port **320** may be positioned on a sidewall near the top of the external vessel **300**. The fitment **345** may also include a hole **340** which enables the external vessel **300** to be placed on a hook and suspended above a surface. While the external vessel **300** is illustrated as having more than one port, embodiments of the present disclosure are not so limited. For example, a single port could be used for both loading and draining of fluid and/or other components from the external vessel **300**. Ports as described herein may permit transfer of fluid and/or other components from outside of the external vessel **300** to the interior compartment of the external vessel **300**, or from the interior compartment of the external vessel **300** to outside of the external vessel **300**, or from one external vessel **300** to another vessel or bag (of the same or different type). Additionally, clamps are depicted in the figures by small rectangles **330** attached to tubing at the ports. Alternatively, valves may be used in place of or in addition to clamps.

In the perfusion system shown in FIG. 3, the structured bag **100** may be a vessel where cells are cultured. The feed vessel **200** may include new media and other reagents which provide nutrients necessary for cell culture and the external vessel **300** may be a waste vessel where old media may be discharged from the structured bag **100**.

Generally, in a perfusion system in accordance with embodiments of the present disclosure, new media may be transferred to a cell culture vessel and old media may be removed from the cell culture vessel while maintaining a closed system through the entire process. As used herein, the term "new media" is used to refer to media that has not previously been used to culture cells. Also as used herein, the term "old media" is used to refer to media that has been used to culture cells for a period of time long enough for the buildup of by-products of cellular metabolism that are toxic to cell growth. As an example, the system may operate to remove old media from the structured bag **100** via the dip tube **110**, through the exit port **160** and to the external vessel **300**. New media may be pumped from the feed vessel **200** into the structured bag **100** through the fluid sampling port **150**. Flow from the feed vessel **200** may be continuous or intermittent by several methods. For example, gravity may be utilized by keeping the fluid level in the feed vessel **200** higher than the fluid level in the structured bag **100**. Where gravity is used, the feed vessel **200** may be placed at a height above the structured bag **100** and gravity allows fluid and/or other components to flow to the structured bag **100**. In such instances, perfusion rate can be controlled based on the height difference or via a valve or other regulated mechanism. As another example, pressurization of the interior of the feed vessel **200** through a vent filter associated with the feed vessel **200** may be utilized to allow fluid and/or other components to flow to the structured bag **100**. Alternatively, the feed vessel **200** may include an inflated cuff configured to be wrapped around the feed vessel **200** in a manner that allows the cuff to apply an external pressure to the feed vessel **200** by squeezing the feed vessel **200**. In another alternative, the feed vessel **200** may include a flexible container housed inside an overpack. A pressurizing gas may be fed to the interstitial space between the flexible container and the overpack to apply a pressure to the flexible container to discharge fluid and/or other components from the flexible container to the structured bag **100**.

Flow to the external vessel **300** may also be continuous or intermittent by the several methods. For example, gravity may be utilized by keeping the fluid level in the structured bag **100** higher than the fluid level in the external vessel **300**. Where gravity is used, the structured bag **100** may be placed at a height above the external vessel **300** and gravity allows fluid and/or other components to flow to the external vessel **300**. In such instances, perfusion rate can be controlled based on the height difference or via a valve or other regulated mechanism. As another example, a vent filter associated with the external vessel **300** may be utilized to generate vacuum conditions in the external vessel **300** to allow fluid and/or other components to flow from the structured bag **100** to the external vessel **300**. Fluid and/or other components will flow from the structured bag **100** to the external vessel **300** until the volumes of the vessels are equal, or until the flow is manually stopped. The perfusion system may include a pump configured to move fluid, and/or other components into and/or out of the structured bag **100**. The pump may be any type of pump including, but not limited to, an infusion pump, a positive displacement pump, a gear pump, a screw pump, a progressive cavity pump, a roots-type pump, a peristaltic pump, a plunger pump, a compressed-air-powered pump, an impulse pump, a velocity pump, and a gravity pump.

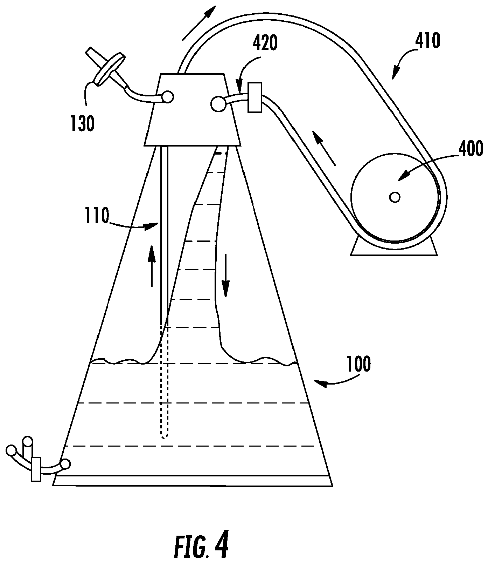

FIG. 4 illustrates a circulation system including a structured bag **100** in accordance with embodiments of the present disclosure. As shown, a structured bag **100** such as the one shown in FIGS. 1A-1B is placed in fluid communication with a circulation tube **410**. A first end of the circulation tube **410** is fluidly connected to a dip tube **110** in the structured bag **100** and a second end of the circulation tube **410** is fluidly connected to a circulation port **420** disposed in the fitment **145** of the structured bag **100**. The circulation port **420** fluidly connects the circulation tube **410** to the interior compartment of the structured bag **100**. The circulation system also includes a pump configured to move fluid and/or other components out of the structured bag **100**, into the circulation system and back into the structured bag **100**.

Generally, in a circulation system in accordance with embodiments of the present disclosure, fluid and/or other components (particularly media) is circulated using the pump **400** to increase oxygenation. Media in the interior compartment of the structured bag **100** is drawn into the circulation tube **410** via the dip tube **110**. Media is then returned to the structured bag **100** through the circulation port **420**. As the media flows into the bag though the circulation port **420**, oxygen diffuses into the media while the media cascades down the bag, for example, down the interior of the sidewall **180** to minimize splashing or disruption of cells in the structured bag **100**. Without wishing to be bound by any particular theory, the cascading of the media functions like a "falling film oxygenator" similar to those used for wine. The circulation system continues when media in the interior compartment of the structured bag **100** is once again drawn into the circulation tube **410** via the dip tube **110**. The circulation system can operate continuously. Alternatively, the circulation system can be configured to operate intermittently. For example, the pump may be manually or automatically operated using a controller to draw media into the circulation system at predetermined instances, for example when certain conditions in the structured bag **100** are sensed, and for predetermined periods of times. The circulation system as described herein draws old media into the circulation tube **410** and returns the media in a manner such that new media is returned to cells in the structured bag **100** while maintaining a closed system through the entire process. According to embodiments of the present disclosure, media may be returned to the structured bag **100** such that cells in the structured bag **100** are relatively undisturbed by controlling the manner, location or rate in which media is returned to the structured bag **100** from the circulation tube **410**.

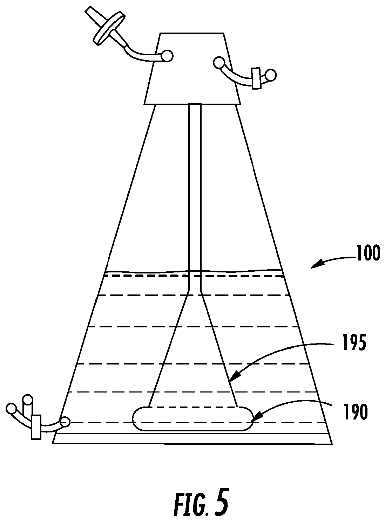

FIG. 5 illustrates a disposable spinner flask including a structured bag in accordance with embodiments of the present disclosure. As shown, the structured bag **100** may include an impeller **195** in the interior compartment of the structured bag **100** which includes a magnetic stir bar **190**. The magnetic stir bar **190** may be connected to a bottom surface of the impeller **195**. Alternatively, the impeller **195** may include a receptacle for receiving the magnetic stir bar **190** molded into a lower portion of the impeller **195**. The structured bag **100** may be placed on a magnetic stir plate which, in combination with the magnetic stir bar **190**, rotates the impeller **195** to stir the fluid and/or other components in the interior compartment of the structured bag **100**.

The disposable spinner flask such as is shown in FIG. 5 may be used to stir cells suspended in a culture media. Stirring in the disposable spinner flask can be performed over a relatively long time (i.e., from several hours up to several months). Stirring in the disposable spinner flask effectively cycles fluid and/or other components from the bottom of the interior compartment to a top surface of the fluid and/or other components, and back again. Typically, cells are maintained at temperatures between about 27°C and about 37°C and are stirred at rates between about 5.0 rpm and about 300 rpm. It should be understood that the temperatures and stirring rates can be varied depending on the particular cells or application.

Optionally, the disposable spinner flask may include microcarriers suspended in cell culture media in the interior compartment to which cells may be attached. The microcarriers may be spheres having diameters of about 125 microns to about 250 microns. Preferably, the size variation of the microcarriers is small to ensure that most, if not all, of the microcarriers can be suspended with gentle stirring. By way of example, the geometric size distribution of the microcarriers may be between about 1.0 and about 1.4. The microcarriers may be solid beads or may be formed from a digestible material. By way of non-limiting example, the microcarriers may be formed from DEAE-dextran, glass, polystyrene plastic, acrylamide, collagen, or alginate. The microcarriers may be coated with proteins, peptides, or charged molecules. While microcarriers are described in relation to the disposable spinner flask of FIG. 5, it should be appreciated that microcarriers are not limited to any single embodiment of the present disclosure and may be included in any of the bags or vessels described herein.

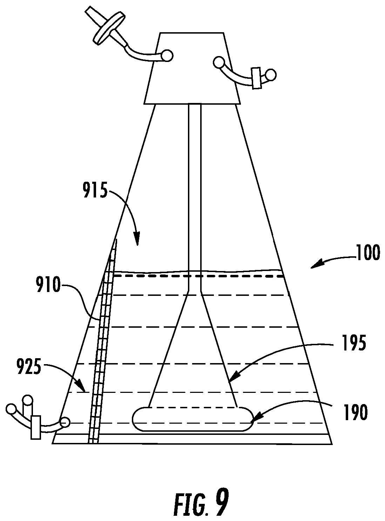

FIG. 9 illustrates a disposable spinner flask (such as the spinner flask of FIG. 5) including a structured bag having a filter material in accordance with embodiments of the present disclosure. As shown, and similar to the disposable spinner flask of FIG. 5, the structured bag **100** may include an impeller **195** in the interior compartment of the structured bag **100** which includes a magnetic stir bar **190**. The structured bag further includes a filter material **910** in the interior compartment. As is shown in FIG. 9, the filter material **910** may be attached to at least one of the sidewall **180** and the bottom portion **102** such that the filter material **910** separates the interior compartment of the structured bag **100** into a first portion **915** and a second portion **925**. Using the disposable spinner flask of FIG. 9 as an example, the first portion **915** of the interior compartment of the structured bag **100** includes the impeller **195** disposed therein and the second portion **925** of the interior compartment is situated such that fluid and/or other components in the second portion **925** are physically closer to the fluid sampling port **150** than fluid and/or other components in the first portion **915**.

As used herein, the term "filter material" is used to describe a porous material including pores having a size through which fluid and target constituents having a size smaller than the pores can pass, but through which other constituents having a size larger than the pores cannot pass. According to embodiments of the present disclosure, the filter material **910** may allow for separation of target constituents of the fluid and/or other components based on size. The target constituents may include, for example, cells, cellular aggregates, particles, particulate aggregates, and molecules. Merely as an example, the filter material **910** may include pores that have a size larger than the size of single cells which is also smaller than the size of spheroids or 3D colonies. By passing fluid containing the single cells through the filter material **910**, the single cells, which are small enough to pass through the pores of the filter material **910**, can be separated from the spheroids or 3D colonies, which are too large to pass through the pores of the filter material **910**. Based on the same principle, the filter material **910** may allow fluid and/or other components to pass therethrough while preventing microcarriers from passing therethrough. The filter material **910** may be formed of a porous material, such as a mesh, netting, perforated sheets, lattice type of material, or any other material that allows target constituents of the fluid and/or other components to pass therethrough while preventing other constituents having a size larger than the pores of the material from passing therethrough. The filter material **910** may have pores in the size of about 15 microns to about 100 microns, or for example, pores having a size of about 30 microns to about 100 microns. It should be appreciated that the size of the pores may be chosen based on the size of the target constituents and/or the other constituents of of the fluid and/or other components. For example, where the first portion **915** of the interior compartment includes microcarriers having diameters of about 125 microns to about 250 microns, the pores of the filter material would have a size of less than about 125 microns to prevent the microcarriers from moving through the filter material **910**. Exemplary materials of the filter material **910** include, but are not limited to, polyethylene terephtalate (PET), polyamide (PA), polypropylene (PP), and polyetheretherketone (PEEK), but also may be polyethylene (PE), polyvinyl chloride (PVC), polystyrene (PS), polycarbonate (PC), polymethylpentene (PMP), polytetrafluoroethylene (PTFE), polyethylene-co-vinyl acetate (EVA), polyfluoroalkoxy (PFA) and derivatives thereof.

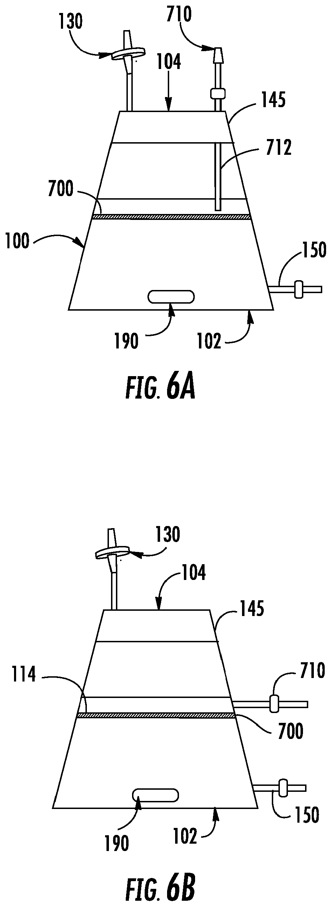

FIGS. 6A-6B illustrate exemplary structured bags including a porous support in accordance with embodiments of the present disclosure. As shown, in addition to a porous support **700**, the exemplary bags also include a feed port **710**, a fluid sampling port **150**, an optional vent port **130**, and an optional stir bar **190**. The porous support **700** is positioned in the interior compartment of the structured bag **100** between the top portion **104** and the bottom portion **102** of the structured bag **100**. The distance between the porous support and the bottom portion **102** of the structured bag **100** may be about 1/8, or about 1/4, or about 1/2 or even about 3/4 of the total height of the structured bag **100**. The structured bags as shown in FIGS. 6A-6B are configured to include a volume of fluid and/or other components above the porous support **700**. Generally, the porous support **700** may be positioned in the structured bag **100** such that oxygen from the air situated above the surface of the fluid and/or other components may diffuse into the fluid and/or other components. Fluid and/or other components may be delivered to the volume above the porous support **700** via the feed port **710**. As shown in FIG. 6A, the feed port **710** may be disposed in the fitment **145** and may be fluidly connected to a tube **712** extending from the feed port **710** and into the volume of media above the porous support **700**. Alternatively, as shown in FIG. 6B, the feed port **710** may be disposed on the sidewall **180** at a portion of the sidewall **180** above the porous support **700**. The porous support **700**, as also shown in FIG. 6B, may include geometric patterns **114**, such as the microwells shown in FIG. 2, that support three dimensional cell growth, such as cell growth into spheroids or 3D colonies.

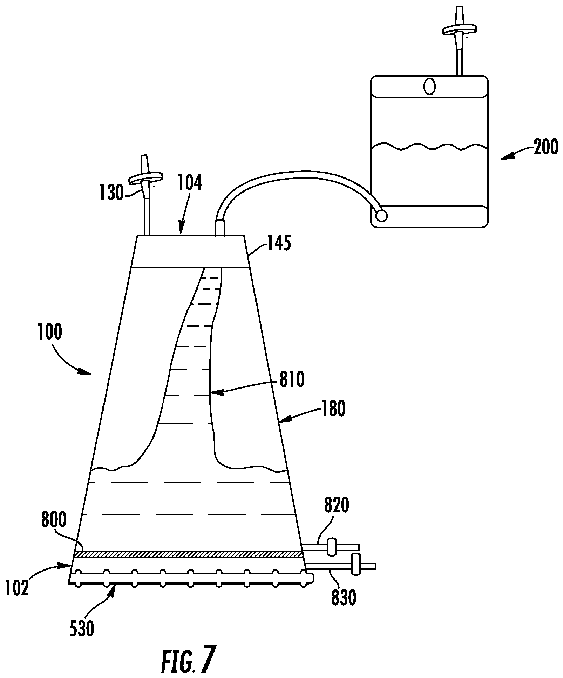

FIG. 7 illustrates a structured bag including an exemplary porous support in accordance with embodiments of the present disclosure. The porous support of the structured bag **100** shown in FIG. 7 is a dialysis membrane **800**. As shown, in addition to a dialysis membrane **800**, the exemplary bag also includes fluid port **820**, fluid port **830**, and an optional vent port **130**. The dialysis membrane **800** is positioned in the interior compartment of the structured bag **100** between the top portion **104** and the bottom portion **102** of the structured bag 100. The dialysis membrane **800** may be positioned near the bottom portion **102** of the bag so as to concentrate cells and cell products in a lower portion of the interior compartment between a lower surface of the dialysis membrane **800** and an upper surface of the bottom portion **102** of the bag. The dialysis membrane **800** is a semi-permeable membrane that includes various sized pores and separates components based on size by allowing components smaller than the pores to pass through the membrane and restricting components larger than the pores from passing through the membrane.

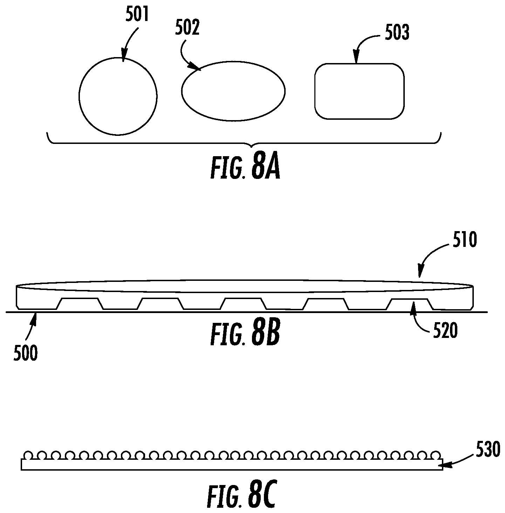

The exemplary structured bag **100** shown in FIG. 7 includes fluid port **820** disposed on the sidewall **180** at a portion of the sidewall **180** above the dialysis membrane **800** and an exit port **830** disposed on the sidewall **180** at a portion of the sidewall **180** below the dialysis membrane **800**. Fluid port **820** is configured to remove fluid and/or other components from an upper portion of the interior compartment between an upper surface of the dialysis membrane **800** and a lower surface of the top portion **104** of the bag, such as a lower surface of the fitment **145**. Fluid port **830** is configured to add or remove fluid and/or other components from a lower portion of the interior compartment between a lower surface of the dialysis membrane **800** and an upper surface of the bottom portion **102** of the bag. According to embodiments of the present disclosure, fluid and/or other components may be provided to the structured bag **100** from a feed vessel **200** through a port in the fitment **145** that is fluidly connected to the feed vessel **200**. The feed vessel **200** may be positioned above the structured bag **100** such that gravity provides the force to deliver the fluid and/or other components to the structured bag **100.** For purposes of illustration, the structured bag **100** of FIG. 7 is shown seated on an exemplary support component **530** which will be discussed in more detail with regards to FIGS. 8A-8C.

According to embodiments of the present disclosure, structured bags as described herein may include support components that assist in allowing the bags to maintain their shape and to be free standing. FIGS. 8A-8C illustrate exemplary support components for a structured bag in accordance with embodiments of the present disclosure. Exemplary shapes of support components, as viewed from the bottom of the support components, are shown in FIG. 8A. Though the support components may be any shape, the shape may be rounded **501**, oval **502**, or rounded rectangular **503**. Support components may be formed from any material, such as a rigid plastic or a metal such as aluminum. Optionally the support component may contact a substantial majority of the bottom portion **102** of the structured bag **100**. Alternatively, the support component may be a ring which contacts the structured bag **100** only around the perimeter of the bottom portion **102** of the bag. Such a ring-shaped support provides increased contact between air and the structured bag **100** and improves air exchange with cells in the bag as compared to a support component that contacts a substantial majority of the bottom portion **102** of the structured bag **100**. FIG. 8B shows an exemplary support component **510** having indentations **520** along a surface. Like the ring-shaped support, the indentations **520** form air channels that facilitate increased contact between air and the structured bag **100** and improve air exchange with cells in the bag as compared to a support component that contacts a substantial majority of the bottom portion **102** of the structured bag **100**. FIG. 8C shows another exemplary support component **530** with geometric protrusions on which the structured bag may sit. Like the indentations **520** shown in FIG. 8B, the protrusions create air spaces that facilitate increased contact between air and the structured bag **100**. According to some embodiments, the support component may prevent the center of the bottom portion **102** of the bag from sagging and causing fluid and/or other components to settle in a high concentration at the center of the interior compartment of the bag.

Embodiments of the present disclosure also relate to kits that include one or more of the structured bags described herein. For example, a kit may include a plurality of structured bags provided together in a package. The bags may be in folded form in the kit. A user may open the packaging and unfold the bags prior to addition of fluid and/or other components. Alternatively, the kits may include fluid and/or other components, wherein the fluid and/or other components are included in the interior compartment of at least one of the plurality of structured bags. Microcarriers as described herein may also be included in the interior compartment of at least one of the plurality of structured bags. The kits may further include any of the system components described in the embodiments herein, such as feed vessels, external vessels, pumps, tubing, etc. According to embodiments of the present disclosure, kits may provide end users with the convenience of easily acquiring parts of a bioprocessing system that are compatible with each other, while limiting the time necessary to assemble the components of the system. The kits may also serve as a turnkey bioprocessing system where implementation simply requires the end user to remove the kit from the packaging.

According to an aspect (1) of the present disclosure, there is provided a structured bag for cell culture according to claim 1.

According to an embodiment (3) of the present disclosure, the structured bag of aspect (2) is provided, wherein the flexible material comprises a plastic film or laminate having high gas permeability.

According to an embodiment (4) of the present disclosure, the structured bag of aspect (2) is provided, wherein the flexible material comprises a film or laminate comprising a metallic layer.

According to an embodiment (5) of the present disclosure, the structured bag of aspect (4) is provided, wherein the metallic layer comprises a metal selected from the group consisting of stainless steel, nickel, chromium, titanium, molybdenum, tungsten, tantalum, niobium, zirconium, gold, tin, and oxides and alloys thereof.

According to an embodiment (7) of the present disclosure, the structured bag of aspect (1) is provided, wherein the bottom portion comprises a rigid substrate.

According to an embodiment (8) of the present disclosure, the structured bag of aspect (7) is provided, wherein the rigid substrate comprises geometric patterns that support three dimensional cell culture.

According to an embodiment (9) of the present disclosure, the structured bag of aspect (8) is provided, wherein the geometric patterns comprise microwells.

According to an embodiment (10) of the present disclosure, the structured bag of any of aspect (1) or embodiments (3) to (5) and (7) to (9) is provided further comprising a dip tube in the interior compartment, wherein the dip tube comprises a proximal end and a distal end, the proximal end being in fluid communication with a port in the fitment and the distal end being in contact with fluid in the interior compartment.

According to an embodiment (11) of the present disclosure, the structured bag of any of aspect (1) or embodiments (3) to (5) and (7) to (9) is provided further comprising an impeller in the interior compartment.

According to an embodiment (12) of the present disclosure, the structured bag of embodiment (11) is provided, wherein the impeller comprises a magnetic stir bar.

According to an embodiment (13) of the present disclosure, the structured bag of any of aspect (1) or the above embodiments is provided further comprising a filter material in the interior compartment.

According to an embodiment (14) of the present disclosure, the structured bag of embodiment (13) is provided, wherein the filter material separates the interior compartment into a first portion and a second portion.

According to an embodiment (15) of the present disclosure, the structured bag of any of embodiments (13)-(14) is provided, wherein the filter material comprises pores having a size of about 15 microns to about 100 microns.

According to an embodiment (13) of the present disclosure, the structured bag of any of embodiments (13)-(15) is provided, wherein the filter material comprises pores in the size of about 30 microns to about 100 microns.

According to an embodiment (17) of the present disclosure, the structured bag of any of aspect (1) or embodiments (3) to (5) or (7) to(9) is provided further comprising a porous support in the interior compartment.

According to an embodiment (18) of the present disclosure, the structured bag of embodiment (17) is provided, wherein the porous support comprises geometric patterns that support three dimensional cell culture.

According to an embodiment (19) of the present disclosure, the structured bag of embodiment (18) is provided, wherein the geometric patterns comprise microwells.

According to an embodiment (20) of the present disclosure, the structured bag of embodiment (17) is provided, wherein the porous support comprises a dialysis membrane.

According to an embodiment (21) of the present disclosure, the structured bag of embodiment (20) is provided, wherein the dialysis membrane is positioned in the interior compartment proximal to the bottom portion.

According to an embodiment (22) of the present disclosure, the structured bag of any of aspect (1) or embodiments (3) to (5) or (7) to (21) is provided, wherein the top portion comprises a fitment.

According to an embodiment (23) of the present disclosure, the structured bag of embodiment (22) is provided, wherein the fitment comprises at least one port.

According to an embodiment (24) of the present disclosure, the structured bag of any of embodiments (22)-(23) is provided, wherein the fitment comprises a hole which enables the structured bag to be suspended above a surface.

According to an embodiment (25) of the present disclosure, the structured bag of any of aspect (1) or embodiments (3) to (5) or (7) to (24) is provided, wherein the bottom portion comprises a bottom panel.

According to an embodiment (26) of the present disclosure, the structured bag of embodiment (25) is provided, wherein the bottom panel comprises an outer edge comprising one or more weights.

According to an embodiment (27) of the present disclosure, the structured bag of any of aspect (1) or embodiments (3) to (5) or (7) to (26) is provided further comprising a vent port.

According to an embodiment (28) of the present disclosure, the structured bag of any of aspect (1) or embodiments (3) to (5) or (7) to (27) is provided further comprising an exit port.

According to an embodiment (29) of the present disclosure, the structured bag of any of aspect (1) or embodiments (3) to (5) or (7) to (28) is provided further comprising a fluid sampling port.

According to an embodiment (30) of the present disclosure, the structured bag of any of aspect (1) or embodiments (3) to (5) or (7) to (29) is provided, wherein the bottom portion is a broad bottom portion.

According to an embodiment (31) of the present disclosure, a perfusion system is provided. The perfusion system comprises: the structured bag of any of aspect (1) or embodiments (3) to (5) or (7) to (30); a feed vessel in fluid communication with the structured bag; and an external vessel in fluid communication with the structured bag.

According to an embodiment (32) of the present disclosure, the perfusion system of aspect (31) is provided, wherein gravity provides the force to deliver fluid to the structured bag.

According to an embodiment (33) of the present disclosure, the perfusion system of aspect (31) is provided, wherein pressurization of the interior of the feed vessel provides the force to deliver fluid to the structured bag.

According to an embodiment (34) of the present disclosure, the perfusion system of aspect (31) is provided, wherein external pressurization of the feed vessel provides the force to deliver fluid to the structured bag.

According to an embodiment (35) of the present disclosure, the perfusion system of any of embodiments (33)-(34) is provided further comprising a pump.

According to an aspect (36) a circulation system is provided. The circulation system comprises; the structured bag of any of aspect (1) or embodiments (3) to (5) and (7) to(30); and a circulation tube having a first end fluidly connected to a dip tube in the interior compartment of the structured bag and a second end fluidly connected to a circulation port in the structured bag.

According to an embodiment (37) of the present disclosure, the circulation system of aspect (36) is provided further comprising a pump configured to move fluid from the dip tube, through the circulation tube, and into the structured bag through the circulation port.

According to an embodiment (38) of the present disclosure, the circulation system of any of aspect (36) or embodiment (37) is provided, wherein fluid cascades from the circulation port down the structured bag.

According to an embodiment (39) of the present disclosure, a kit is provided. The kit comprises a plurality of the structured bag of any of aspect (1) or embodiments (3) to (5) and (7) to (30) in a package.

## Claims

1. A structured bag for cell culture comprising:
a top portion;
a bottom portion having a shape; and
a sidewall extending between the top portion and the bottom portion and at least partially defining an interior compartment for receiving fluid,
wherein the bottom portion is configured to maintain the shape to support the structured bag such that the structured bag is free standing when placed on a surface, and
**characterised in that** the bottom portion and at least one of the top portion and the sidewall is formed from a flexible material, and wherein the flexible material of the bottom portion is thicker than the flexible material of the at least one of the top portion and the sidewall.

2. The structured bag of claim 1, wherein the flexible material comprises a plastic film or laminate having gas permeability.

3. The structured bag of claim 1, wherein the bottom portion comprises a rigid substrate.

4. The structured bag of claim 3, wherein the rigid substrate comprises geometric patterns that support three-dimensional cell culture.

5. The structured bag of any of the preceding claims, further comprising a dip tube in the interior compartment,
wherein the top portion comprises a fitment, and
wherein the dip tube comprises a proximal end and a distal end, the proximal end being in fluid communication with a port in the fitment and the distal end being in contact with fluid in the interior compartment.

6. The structured bag of any of claims 1-4 further comprising an impeller in the interior compartment.

7. The structured bag of any of the preceding claims, further comprising a filter material in the interior compartment.

8. The structured bag of Claim 7, wherein the filter material separates the interior compartment into a first portion and a second portion.

9. The structured bag of any of claims 1-4 further comprising a porous support in the interior compartment.

10. The structured bag of claim 9, wherein the porous support comprises geometric patterns that support three-dimensional cell culture.

11. The structured bag of claim 9, wherein the porous support comprises a dialysis membrane.

12. The structured bag of any of the preceding claims, wherein the bottom portion comprises a bottom panel.

13. The structured bag of any of the preceding claims, wherein the bottom portion has at least one dimension that is larger than the same or a similar dimension of the top portion.

## Patentansprüche

1. Strukturierter Beutel für Zellkultur, umfassend:
einen oberen Teil;
einen unteren Teil mit einer Form; und
eine Seitenwand, die sich zwischen dem oberen Teil und dem unteren Teil erstreckt und wenigstens teilweise eine innere Kammer zur Aufnahme von Fluid definiert;
wobei der untere Teil so gestaltet ist, dass er die Form hält, um den strukturierten Beutel zu stützen, so dass der strukturierte Beutel frei steht, wenn er auf einer Oberfläche platziert ist; und
**dadurch gekennzeichnet, dass** der untere Teil und wenigstens der obere Teil und/oder die Seitenwand aus einem flexiblen Material besteht, und wobei das flexible Material des unteren Teils dicker ist als das flexible Material des wenigstens einen aus dem oberen Teil und/oder der Seitenwand.

2. Strukturierter Beutel nach Anspruch 1, wobei das flexible Material einen Kunststofffilm oder ein Laminat mit Gasdurchlässigkeit umfasst.

3. Strukturierter Beutel nach Anspruch 1, wobei der untere Teil ein starres Substrat umfasst.

4. Strukturierter Beutel nach Anspruch 3, wobei das starre Substrat geometrische Muster umfasst, die eine dreidimensionale Zellkultur stützen.

5. Strukturierter Beutel nach einem der vorstehenden Ansprüche, der ferner ein Tauchrohr in der inneren Kammer umfasst,
wobei der obere Teil einen Verbau umfasst, und
wobei das Tauchrohr ein proximales Ende und ein distales Ende umfasst, wobei sich das proximale Ende in Fluidkommunikation mit einem Anschluss in dem Verbau befindet, und wobei sich das distale Ende in Kontakt mit dem Fluid in der inneren Kammer befindet.

6. Strukturierter Beutel nach einem der Ansprüche 1 bis 4, der ferner ein Flügelrad in der inneren Kammer umfasst.

7. Strukturierter Beutel nach einem der vorstehenden Ansprüche, der ferner ein Filtermaterial in der inneren Kammer umfasst.

8. Strukturierter Beutel nach Anspruch 7, wobei das Filtermaterial die innere Kammer in einen ersten Teil und einen zweiten Teil trennt.

9. Strukturierter Beutel nach einem der Ansprüche 1 bis 4, der ferner eine poröse Stütze in der inneren Kammer umfasst.

10. Strukturierter Beutel nach Anspruch 9, wobei die poröse Stütze geometrische Muster umfasst, die eine dreidimensionale Zellkultur stützen.

11. Strukturierter Beutel nach Anspruch 9, wobei die poröse Stütze eine Dialysemembran umfasst.

12. Strukturierter Beutel nach einem der vorstehenden Ansprüche, wobei der untere Teil eine untere Platte umfasst.

13. Strukturierter Beutel nach einem der vorstehenden Ansprüche, wobei der untere Teil mindestens eine Abmessung aufweist, die größer ist als die gleiche oder eine ähnliche Abmessung des oberen Teils.

## Revendications

1. Sac structuré pour la culture cellulaire comprenant :
une partie supérieure ;
une partie inférieure ayant une forme ; et
une paroi latérale s'étendant entre la partie supérieure et la partie inférieure et définissant au moins partiellement un compartiment intérieur destiné à recevoir du fluide,
la partie inférieure étant conçue pour conserver la forme pour soutenir le sac structuré de sorte que le sac structuré soit libre de se tenir debout lorsqu'il est placé sur une surface, et
**caractérisé en ce que** la partie inférieure et la partie supérieure et/ou la paroi latérale sont formées d'un matériau flexible, et le matériau flexible de la partie inférieure étant plus épais que le matériau flexible de la partie supérieure et/ou de la paroi latérale.

2. Sac structuré selon la revendication 1, le matériau flexible comprenant un film plastique ou un stratifié ayant une perméabilité aux gaz.

3. Sac structuré selon la revendication 1, la partie inférieure comprenant un substrat rigide.

4. Sac structuré selon la revendication 3, le substrat rigide comprenant des motifs géométriques qui soutiennent la culture cellulaire tridimensionnelle.

5. Sac structuré selon l'une quelconque des revendications précédentes, comprenant en outre un tube plongeur dans le compartiment intérieur,
la partie supérieure comprenant un accessoire, et
le tube plongeur comprenant une extrémité proximale et une extrémité distale, l'extrémité proximale étant en communication fluidique avec un orifice dans l'accessoire et l'extrémité distale étant en contact avec le fluide dans le compartiment intérieur.

6. Sac structuré selon l'une quelconque des revendications 1 à 4, comprenant en outre une roue dans le compartiment intérieur.

7. Sac structuré selon l'une quelconque des revendications précédentes, comprenant en outre un matériau filtre dans le compartiment intérieur.

8. Sac structuré selon la revendication 7, le matériau filtre séparant le compartiment intérieur en une première partie et une seconde partie.

9. Sac structuré selon l'une quelconque des revendications 1 à 4 comprenant en outre un support poreux dans le compartiment intérieur.

10. Sac structuré selon la revendication 9,
le support poreux comprenant des motifs géométriques qui permettent la culture de cellules en trois dimensions.

11. Sac structuré selon la revendication 9, le support poreux comprenant une membrane de dialyse.

12. Sac structuré selon l'une quelconque des revendications précédentes, la partie inférieure comprenant un panneau inférieur.

13. Sac structuré selon l'une quelconque des revendications précédentes, la partie inférieure ayant au moins une dimension qui est plus grande que la même dimension ou une dimension similaire de la partie supérieure.
